# EUROPÄISCHE PATENTSCHRIFT

(11) **EP 2 063 772 B1**
(45) Veröffentlichungstag und Bekanntmachung des Hinweises auf die Patenterteilung: **31.08.2011**
(21) Anmeldenummer: 08827015.2
(22) Anmeldetag: 07.11.2008
(51) Int. Cl.: A61B 5/1455

(54) **VORRICHTUNG ZUR MESSUNG DER SAUERSTOFFSÄTTIGUNG IM BLUT**
APPARATUS FOR MEASURING BLOOD OXYGEN SATURATION
DISPOSITIF DE MESURE DE LA SATURATION EN OXYGÈNE DU SANG

(30) Priorität: 09.11.2007 DE 102007053599
(43) Veröffentlichungstag der Anmeldung: 03.06.2009
(73) Patentinhaber: Envitec-Wismar GmbH, 23966 Wismar (DE)
(72) Erfinder: ECKERMANN, Martin, 18055 Rostock (DE); SCHOLL, Thomas, 23968 Wismar (DE)
(74) Vertreter: Teipel, Stephan
(86) Internationale Anmeldenummer: PCT/EP2008/009429
(87) Internationale Veröffentlichungsnummer: WO 2009/019044

(56) Entgegenhaltungen:
- EP-A- 0 227 401
- DE-A1-102005 053 623
- US-A- 3 017 885
- US-A- 5 701 155
- US-A1- 2007 027 375

## Beschreibung

Die Erfindung betrifft eine Vorrichtung zur Messung der Sauerstoffsättigung im Blut. Sie betrifft ferner ein Verfahren zur Herstellung einer derartigen Vorrichtung sowie Verwendungen der Vorrichtung.

Die Messung der Sauerstoffsättigung im Blut ist ein in der Medizin weitverbreitetes Verfahren, wobei eine derartige Messung in der Mehrzahl der Fälle nicht-invasiv mittels eines Pulsoxymeters vorgenommen wird. Ein Pulsoxymeter besteht in der Regel aus einem clipartigen Gehäuse mit einer Ausnehmung, in die beispielsweise der Finger, eine Zehe, das Ohrläppchen oder andere durchblutete Gewebe einer Person eingeführt werden können. In der Oberfläche der Ausnehmung befindet sich eine Lichtquelle, der ein ebenfalls in der Oberfläche der Ausnehmung befindlicher Detektor derart gegenüberliegt, das sich das durchblutete Gewebe zwischen der Lichtquelle und dem Detektor befindet. Die Lichtquelle, normalerweise eine LED, emittiert nur Licht mit zwei unterschiedlichen Wellenlängen, beispielsweise 660 nm und 940 nm. Das durchstrahlte durchblutete Gewebe absorbiert einen Teil des Lichtes, wobei die Lichttransmission umgekehrt proportional zur Konzentration des Hämoglobins ist. Die Lichtquelle und der Detektor bilden das als Sensor bezeichnete Element des Pulsoxymeters. Eine entsprechende Vorrichtung ist beispielsweise in EP-A-1 168 959 oder US 2007/0027375 A1 beschrieben.

Der Detektor, üblicherweise ein Photodetektor, empfängt das transmittierte Licht und wandelt dieses in elektrische Signale um. Die elektrischen Signale werden mittels elektrischer Leiter, die in dem Gehäuse ausgebildet sind, über ein Verbindungskabel zu einer Auswerteeinheit transportiert. Zu diesem Zweck weist das Kabel einen Anschlußstecker auf, der in ein entsprechendes Anschlußelement in der Auswerteeinheit eingesteckt werden kann, so daß eine elektrische Verbindung zwischen dem Detektor und der Auswerteinheit hergestellt wird. Anderseits werden durch das Kabel elektrische Leiter für die Energieversorgung der Lichtquelle und des Detektors geführt.

Es ist medizinischer Standard, medizinische Geräte regelmäßig zu sterilisieren, um eine möglichst weitgehende Keimfreiheit der Geräte zu gewährleisten. Es ist inzwischen üblich, die Sterilisierung mittels Autoklavieren durchzuführen. Dazu wird das zu sterilisierende Geräte in einen Autoklaven eingebracht und dort typischerweise etwa 2 bis 20 min oder sogar länger bei einer Temperatur von 115 °C bis 140 °C und einem Druck von etwa 2 bar gesättigtem Wasserdampf ausgesetzt.

Medizinische Geräte bestehen im Regelfall aus mehreren Einzelelementen, die auf vielfache und unterschiedliche Weise wie mechanisch, elektrisch, optisch, magnetisch etc. und/oder deren Kombinationen gekoppelt sind und in einer funktionsrealisierenden Wirkverbindung miteinander stehen. Mechanisch trennbare Verbindungen sind insbesondere erforderlich, um Elemente des medizinischen Gerätes, die nicht sterilisierbar sind, abgekoppelt und einem anderen Sterilisationsprozeß als dem Autoklavieren unterziehen zu können. Dementsprechend werden in DE 91 02 784 U1 Kopplungselemente vorgeschlagen, mit deren Hilfe ein Temperatursensor von dem Verbindungskabel getrennt werden kann, wodurch eine Autoklavierung des Temperatursensors ermöglicht wird. Das Verbindungskabel selbst wird entweder nicht sterilisiert oder einer einfachen Wischsterilisation unterzogen.

Zur hinreichend sicheren Sterilisation des vorstehend beschriebenen Pulsoxymeters muß somit das Verbindungskabel zunächst von der Auswerteeinheit getrennt werden, indem der Anschlußstecker aus der Anschlußeinheit der Auswerteinrichtung gezogen wird. Anschließend werden das Gehäuse, der die Lichtquelle und den Detektor umfassende Sensor, das Verbindungskabel und der Anschlußstecker, die schlüssig miteinander verbunden sind und das eigentliche Meßgerät zur Bestimmung der Sauerstoffsättigung von Blut bilden, voneinander getrennt, so daß vier Einzelelemente erhalten werden: Gehäuse, Sensor, Kabel und Anschlußstecker. Die Zerlegung in diese Einzelelemente ist erforderlich, da bestimmte Einzelelemente (wie beispielsweise das Verbindungskabel sowie der Sensor) dem Autoklavieren nicht unterzogen werden können oder zumindest das Überstülpen eines Hohlkörpers, der aus einem hochtemperaturfesten Kunststoff besteht, erfordern. Mittels des Hohlkörpers soll eine Beschädigung des Einzelelementes während des Autoklavierens durch Überhitzung verhindert werden. Kann ein Einzelelement nicht autoklaviert werden, so muß ein anderes Sterilisationsverfahren verwendet werden.

Zudem können herkömmliche Vorrichtungen zur Messung der Sauerstoffsättigung im Blut nicht als ein Stück autoklaviert werden, da durch den hohen Druck von etwa 2 bar leicht Wasserdampf in die elektronischen Bauteile eindringen und die Vorrichtung dadurch zerstören kann. Ein weiteres Problem besteht darin, dass die benötigten optischen Halbleiter-Bauelemente, die in der Regel als Sensor eine LED und als Empfänger eine Si-Photodiode umfassen, oftmals nicht genügend temperaturbeständig sind, um den hohen Temperaturen beim Autoklavieren standzuhalten. Der Fachmann war daher davon abgehalten, einstückige Vorrichtungen der genannten Art bereitzustellen.

Die Zerlegung des Meßgerätes in seine vier Einzelelemente ist jedoch mit mehreren Nachteilen verbunden. Es ist zum einen zeitaufwendig, da einerseits eine Demontage und Montage des Meßgerätes erforderlich und anderseits Einzelteile unterschiedlichen Sterilisationsverfahren ausgesetzt werden müssen, wozu entsprechende Vorrichtungen erforderlich sind. Zum anderen ist es fehleranfällig, da erstens bei der Montage der Einzelelemente Fehler gemacht werden können und zweitens sterilisierte und nichtsterilisierte Einzelelemente zusammengesetzt werden können.

Eine Aufgabe der Erfindung ist es daher, die Nachteile der Vorrichtungen nach dem Stand der Technik zu überwinden. Es soll insbesondere eine Vorrichtung zur Messung der Sauerstoffsättigung im Blut angegeben werden, die eine Sterilisierung ermöglicht, ohne daß eine Zerlegung der Vorrichtung in seine Einzelelemente erforderlich ist. Ferner sollen ein Verfahren zur Herstellung einer derartigen Vorrichtung sowie Verwendungen der Vorrichtung angegeben werden.

Diese Aufgabe wird durch die Merkmale der Ansprüche 1, 11 und 13 gelöst. Zweckmäßige Ausgestaltungen der Erfindung ergeben sich aus den Merkmalen der Ansprüche 2 bis 10, 12, 14 und 15.

Nach Maßgabe der Erfindung ist eine Vorrichtung zur Messung der Sauerstoffsättigung im Blut vorgesehen, die ein Gehäuse, einen Sensor, ein Verbindungskabel und einen Anschlußstecker umfaßt, wobei
- das Gehäuse eine Ausnehmung zur Aufnahme von durchblutetem Gewebe eines Patienten aufweist;
- der Sensor, der an dem Gehäuse angeordnet ist und zumindest eine Lichtquelle zur Emission von Licht, das das durchblutete Gewebe durchdringt, und einen Detektor zum Empfang des durch das durchblutete Gewebe hindurchdringenden Lichtes aufweist, wobei es sich bei der Lichtquelle und dem Detektor um optische Halbleiter-Bauelemente handelt;
- die Vorrichtung über den Anschlußstecker mit einer Auswerteeinheit verbunden werden kann;
- das Gehäuse, der Sensor, das Verbindungskabel und der Anschlußstecker schlüssig und unlösbar miteinander verbunden sind;
- die Außenseiten zumindest des Gehäuses, des Verbindungskabels und des Anschlußsteckers aus einem ersten flexiblen, autoklavierbaren Material gebildet sind; und
- Lichtquelle und Detektor so ausgewählt sind, dass sie die benötigte Temperaturstabilität aufweisen, um den hohen Temperaturen beim Autoklavieren standzuhalten.

Die erfindungsgemäße Vorrichtung kann ohne Demontage in ihrer Einzelelemente in einem Autoklaven sterilisiert werden, da das Verbindungskabel und der Anschlußstecker aufgrund des flexiblen, autoklavierbaren Materials, das ihre Außenseite bildet, autoklaviert werden können. Durch die nachfolgend beschriebenen Maßnahmen können darüber hinaus auch Gehäuse und Sensor dergestalt zur Verfügung gestellt werden, dass auch der Sensor beschädigungsfrei autoklaviert werden kann. Eine Montage der Einzelelemente nach der Sterilisation ist somit ebenfalls nicht mehr erforderlich. Ebenso werden Fehler bei der Demontage bzw. Montage und bei der gesonderten Sterilisation der Einzelelemente vermieden.

Die erfindungsgemäße Vorrichtung ermöglicht die Sterilisation in einem einzigen Verfahrensschritt, was die Handhabung der Vorrichtung erheblich vereinfacht und die Sterilisation beschleunigt. Eine zeitaufwendige Vor- und Nachbearbeitung der Vorrichtung im Zusammenhang mit der Autoklavierung ist nicht erforderlich, was einen weiteren Zeitgewinn bedeutet.

Unter dem Begriff "Außenseite" wird in der vorliegenden Erfindung jede Oberfläche verstanden, die mit der Umgebung in Kontakt kommt und damit mit biologischen Keimen kontaminiert sein kann. Oberflächen, die nicht der Umgebung ausgesetzt sind, beispielsweise die Innenwand des geschlossenen Gehäuses oder die den elektrischen Leitern des Verbindungskabels zugewandte Seite des Isoliermaterials des Verbindungskabels sollen nicht als Außenseite angesehen werden, wobei jedoch das Gehäuse, das Isoliermaterial des Verbindungskabels und das Gehäuse des Anschlußsteckers vollständig aus dem ersten flexiblen, autoklavierbaren Material gebildet sein können. Die elektrischen Kontakte des Anschlußsteckers, die aus einem elektrisch leitfähigen Metall gebildet sind und in die die elektrischen Kontakte des Anschlußelementes der Auswerteinrichtung eingreifen können, weisen keine Außenseiten aus dem ersten flexiblen, autoklavierbaren Material auf, sondern liegen frei, da sie ohnehin aus einem autoklavierbaren Material, einem Metall, bestehen.

Unter dem Begriff "autoklavierbares Material" wird ein Material verstanden, das durch die Sterilisierung im Autoklaven hinsichtlich seiner chemischen und physikalischen Eigenschaften nicht verändert wird. Das autoklavierbare Material sollte daher bei Temperaturen bis zu 180°C wärmebeständig sein und sich bei der Einwirkung von gesättigtem Wasserdampf nicht verändern, beispielsweise lösen oder zersetzen.

Unter einem "flexiblen Material" wird ein elastisches Material verstanden, das nach einer Deformation unter der Einwirkung einer Kraft nach dem Wegfall der Kraft in seinen ursprünglichen Zustand zurückkehrt. Bevorzugte flexible Materialien sind gummielastische Materialien.

Das flexible, autoklavierbare Material ist vorzugsweise ein Kunststoff, stärker bevorzugt ein Elastomer, besonders bevorzugt ein Silicon.

In einer Ausführungsform der Erfindung weisen das Gehäuse, das Verbindungskabel und/oder der Anschlußstecker eine Beschichtung aus dem ersten flexiblen, autoklavierbaren Material auf, wobei diese Beschichtung jeweils die Außenseite des Gehäuses, des Verbindungskabels und/oder des Anschlußsteckers bildet.

Vorzugsweise bestehen die Außenseiten des Sensors aus einem zweiten autoklavierbaren Material, wobei das zweite autoklavierbare Material dasselbe oder ein anderes als das erste flexible, autoklavierbare Material sein kann. Das zweite autoklavierbare Material muß für das vom Sensor emittierte Licht durchlässig sein. Vorzugsweise ist das zweite autoklavierbare Material ebenfalls ein flexibles, autoklavierbares Material.

Da die Sensorik des Sensors in der Regel wärmeempfindlich ist, ist die Sensorik normalerweise ohnehin von einem Material umschlossen, das selbst autoklavierbar ist oder eine autoklavierbare Beschichtung beispielsweise in Form eines Überzuges aufweist. Alternativ kann der Sensor in das Gehäuse integriert sein, so daß die dem durchbluteten Gewebe zugewandten Außenseiten des Sensors von dem Gehäuse gebildet werden. In diesem Fall muß das erste flexible autoklavierbare Material für das emittierte Licht durchlässig sein.

In einer bevorzugten Ausführungsform sind das Gehäuse, das Verbindungskabel, der Anschlußstecker und gegebenenfalls der Sensor vollständig in einen gemeinsamen Überzug aus dem ersten flexiblen autoklavierbaren Material eingeschlossen. Dabei bildet das erste flexible autoklavierbare Material eine Ummantelung für das Gehäuse, den Sensor, das Verbindungskabel und den Anschlußstecker.

Eine solche Ummantelung kann auch nachträglich bei bereits vorhandenen Vorrichtungen, die aus den Einzelelementen Gehäuse, Sensor, Verbindungskabel und Anschlußstecker bestehen, hergestellt werden.

Es wurde darüber hinaus überraschend gefunden, dass die bekannten Probleme durch die mangelnde Temperaturbeständigkeit optischer Halbleiter-Bauelemente durch die gezielte Auswahl hochtemperaturstabiler Bauelemente gelöst werden können. Hierzu können beispielsweise aus einer herkömmlichen Charge entsprechender Bauelemente diejenigen ausgewählt werden, die die benötigte Temperaturstabilität aufweisen.

Außerdem hat es sich als vorteilhaft erwiesen, wenn Sender/Lichtquelle und Empfänger/Detektor auf einen Keramikträger aufgebracht und mit einem für das emittierte Licht durchlässigen, autoklavierbaren Material umschlossen, vorzugsweise darin eingegossen sind. Hierdurch wird die Resistenz gegen den heißen Wasserdampf und den hohen Druck verbessert.

Beispielsweise können die Kontaktflächen (Leiterbahnen) mit einem temperaturbeständigen Klebstoff auf einen Keramikkörper aufgebracht werden. Die Halbleiterbauelemente können dann mit ihren Unterseiten mit einem temperaturbeständigen Leitkleber auf die Leiterbahn aufgebracht werden. Anschließend werden die Halbleiterbauelemente auf ihrer Oberseite mit einem Bonddraht zur zweiten Leiterbahn kontaktiert und die Halbleiterbauelemente sowie der Bonddraht werden mit einem transparenten, temperatur- und druckstabilen, wasserdampfresistenten Vergussmaterial komplett gekapselt.

Bei dem speziellen, vorstehend beschriebenen Aufbau von Sensor und Empfänger ist darauf zu achten, dass das transparente Vergussmaterial ausreichend temperaturbeständig ist. Die hohe Temperatur beim Autoklavieren führt sonst zu Verfärbungen oder Eintrübungen des Vergussmaterials. Eine Eintrübung des Materials würde zu einer maßgeblichen Verschlechterung oder sogar zu einem kompletten Ausfall der Sensoreigenschaften führen. Darüber hinaus ist bei der Auswahl aller eingesetzten Materialien darauf zu achten, dass diese zumindest an den Kontaktflächen ähnliche Temperaturausdehnungskoeffizienten besitzen, um zu vermeiden, dass sich bei hohen Temperaturen die Elemente von einander lösen. Besonders beim Halbleiter-Detektor spielen die Temperaturausdehnungskoeffizienten eine entscheidende Rolle, da dieser eine große Kontaktfläche von 3-8 mm² zur Leiterbahn aufweist.

Die bekannten Probleme beim Autoklavieren der Sensoren in Vorrichtungen zur Messung der Sauerstoffsättigung im Blut können erfindungsgemäß somit durch drei Maßnahmen überwunden werden, die einzeln oder in beliebiger Kombination miteinander eingesetzt werden können. Erstens können Lichtquelle und Detektor auf einen Keramikträger aufgebracht werden, um so die Temperatur- und Druckstabilität zu erhöhen. Zweitens können Lichtquelle und Detektor einschließlich ihres Trägers, vorzugsweise des Keramikträgers, mit einem transparenten Vergussmaterial eingekapselt werden. Drittens ist es vorteilhaft, die für den Aufbau von Lichtquelle und Detektor verwendeten Materialien so zu wählen, dass sie möglichst geringe Unterschiede in ihren Temperaturausdehnungskoeffizienten aufweisen.

Weiter vorteilhaft handelt es sich bei dem Gehäuse um ein Silicon-Gehäuse mit eingespritzten, transparenten Silicon-Fenstern, unter denen Lichtquelle und Sensor angeordnet sind. Unter transparenten Silicon-Fenstern werden dabei solche Fenster verstanden, die für das emittierte Licht durchlässig sind. Durch das Einspritzen der Silicon-Fenster in das Silicon-Gehäuse wird ein besonderer Schutz der elektronischen Bauteile gegen den heißen Wasserdampf auch bei hohen Temperaturen gewährleistet.

Schließlich hat es sich als vorteilhaft erwiesen, wenn der Anschlussstecker an das Verbindungskabel angespritzt wird, vorzugsweise mit einem geeigneten temperaturfesten Material.

Ferner sieht die Erfindung ein Verfahren vor, das
- das Bereitstellen eines Gehäuses, eines Sensors, eines Verbindungskabels und eines Anschlußsteckers, wobei zumindest einer dieser Bestandteile eine Außenseite aus einem nicht hitzebeständigen Material aufweist; und
- das Besprühen der Außenseiten der Bestandteile mit einer autoklavierbaren Substanz;
umfaßt, wobei die autoklavierbare Substanz eine gel- oder schaumartige Konsistenz aufweist oder sich im gasförmigen Zustand befindet, so daß die autoklavierbare Substanz in die Oberflächen der Bestandteile unter Ausbildung der Außenseiten aus dem ersten flexiblen, autoklavierbaren Material diffundiert.

Die autoklavierbare Substanz, die nach dem Auftragen auf die Bestandteile der Vorrichtung deren Außenseiten aus dem flexiblen, autoklavierbare Material darstellt, bildet somit eine Beschichtung aller Bestandteile der Vorrichtung. Die Beschichtung besteht aus dem ersten flexiblen, autoklavierbaren Material. Diese Beschichtung aus dem ersten flexiblen, autoklavierbaren Material stellt vorzugsweise eine Ummantelung der gesamten Vorrichtung dar.

Als autoklavierbare Substanz kann das erste flexible autoklavierbare Material verwendet werden. Es ist jedoch auch möglich, das die autoklavierbare Substanz erst nach dem Auftragen auf die Bestandteile der Vorrichtung das flexible autoklavierbare Material ausbildet.

Der Begriff "Bestandteile" bezieht sich auf das Gehäuses, den Sensor, das Verbindungskabel und den Anschlußstecker der Vorrichtung. Die Bestandteile sind schlüssig miteinander verbunden. Nach dem Auftragen der autoklavierbaren Substanz sind die Bestandteile nicht mehr voneinander in Einzelelemente trennbar.

Die erfindungsgemäße Vorrichtung wird vorteilhaft zur Messung der Sauerstoffsättigung im Blut, insbesondere des SₚO₂-Wertes verwendet. Sie ist insbesondere zur kontinuierlichen Überwachung der Sauerstoffsättigung geeignet.

Die Erfindung wird nachstehend anhand eines Beispiels unter Bezugnahme auf die Zeichnungen näher erläutert. Dabei zeigen
- Fig. 1: eine Draufsicht auf eine Ausführungsform der erfindungsgemäßen Vorrichtung;
- Fig. 2: eine Schnittansicht der in Fig. 1 gezeigten Ausführungsform;
- Fig.3: eine Detailansicht des in Fig. 1 gezeigten Gehäuses der ersten Ausführungsform;
- Fig.4: eine Detailansicht des in Fig.2 gezeigten Gehäuses der ersten Ausführungsform; und

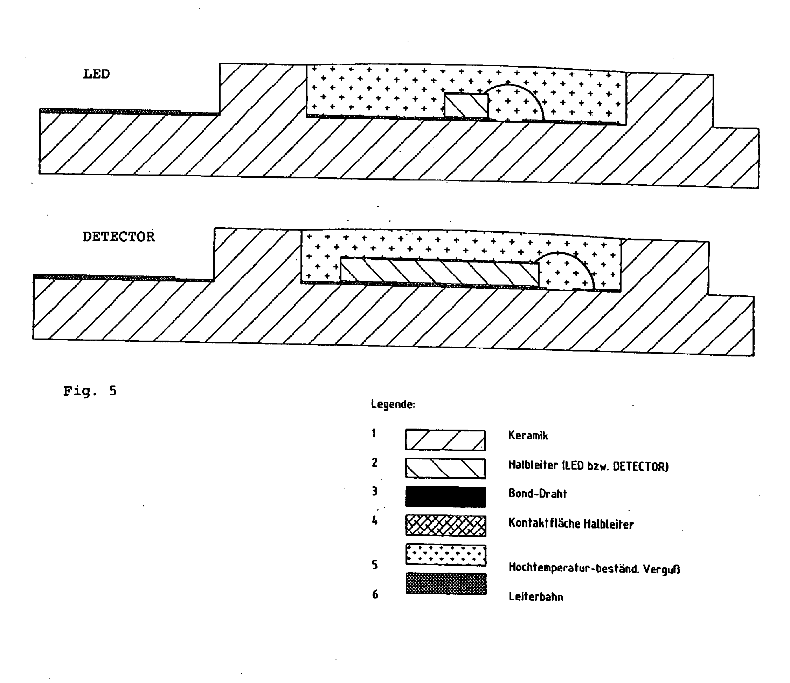

- Fig. 5: schematische Aufbauten von einer bevorzugten Lichtquelle (LED) und einem bevorzugten Detekor.

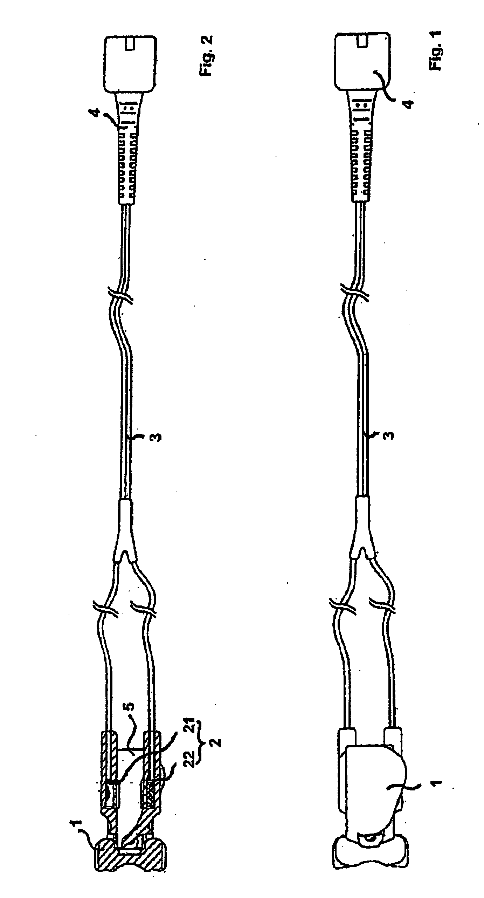

Nach den Figuren 1 und 2 weist die erfindungsgemäße Vorrichtung ein Gehäuse 1, einen Sensor 2, ein Verbindungskabel 3 und einen Anschlußstecker 4 auf. In dem Gehäuse 1 ist der Sensor 2, der eine Lichtquelle 21 und einen Detektor 22 umfaßt, derart angeordnet, das Licht, das von der Lichtquelle 21 emittiert wird, nach der Transmission durch ein durchblutetes Gewebe eines Patienten, von dem Detektor 22 empfangen wird.

Das Gehäuse 1 ist schlüssig mit einem ersten Ende des Verbindungskabels 3 verbunden, wobei das zweite Ende des Verbindungskabels 3 schlüssig mit dem Anschlußstecker 4 verbunden ist. In dem Verbindungskabel 3 verlaufen die elektrische Leiter (nicht gezeigt), die die Lichtquelle 21 und den Detektor 22 des Sensors 2 in dem Gehäuse 1 mit den Polen des Anschlußsteckers 4 verbinden, die in entsprechende elektrische Kontakte eines Anschlußelementes der Auswerteeinheit (nicht gezeigt) eingeführt werden können.

Das Gehäuse 1, der Sensor 2, das Verbindungskabel 3 und der Anschlußstecker 4 sind an ihren Außenseiten mit Silicon beschicht, so daß alle Bestandteile der Vorrichtung mit einem flexiblen, autoklavierbaren Material überzogen sind. Insbesondere ist auch die Ausnehmung 5 des Gehäuses 1, in das das durchblutete Gewebe des Patienten eingeführt wird, von dem flexiblen, autoklavierbaren Material überzogen.

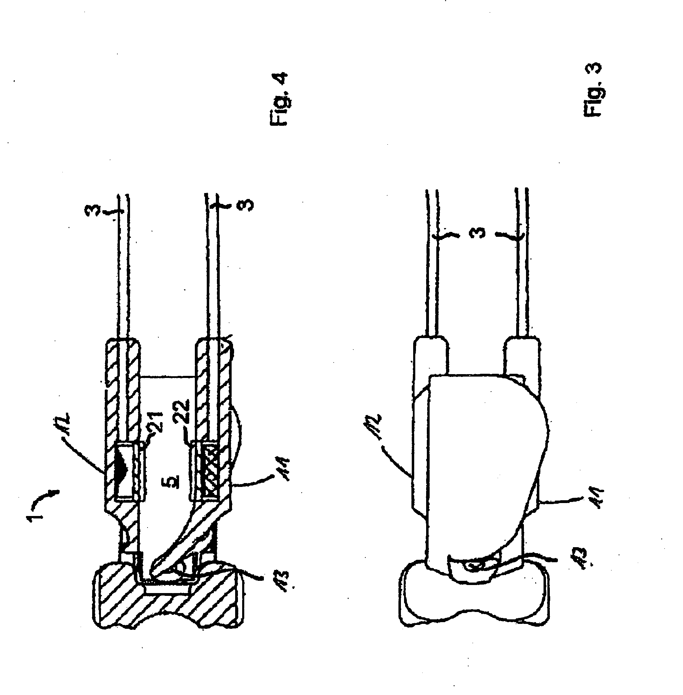

Nach den Figuren 3 und 4 weist das Gehäuse 1 beispielhaft eine Grundplatte 11 und eine Deckplatte 12, die von der Grundplatte 11 beabstandet ist und in einer Ebene, die parallel zu der Grundplatte 11 verläuft, verdrehbar ist, auf. Ein Abschnitt der Grundplatte 11 ist als Auflage für das durchblutete Gewebe, beispielsweise einen Finger, ausgeformt. An den Längsseiten der Grundplatte 11 sind ein erstes Seitenelement und ein zweites Seitenelement angeordnet, während an einer Schmalseite der Grundplatte 11 ein drittes Seitenelement angeordnet ist. Die Seitenelemente erstrecken sich senkrecht zur Grundplatte 11 bis zur Deckplatte 12. Dabei umgrenzen die Seitenelemente gemeinsam mit der Grundplatte 11 und der Deckplatte 12 die Ausnehmung 5 zur Aufnahme des durchbluteten Gewebes, wobei die Ausnehmung 5 nach unten hin von der Grundplatte 11 und nach oben hin von der Deckplatte 12 abgeschlossen wird. Zwischen den Schmalseiten der Grundplatte 11 und Deckplatte 12, die dem dritten Seitenelement abgewandt sind, ist die Ausnehmung 5 zur Umgebung hin geöffnet.

Die Grundplatte 11 und die Deckplatte 12 weisen jeweils einen Knickschutz für das Kabel 3 auf.

Ferner weist das Gehäuse ein Federelement 13 auf, das an dem dritten Seitenelement und an der Grundplatte 11 andererseits befestigt ist. Das Federelement 13 spannt die Grundplatte 11 in Richtung der Deckplatte 12. Aufgrund dieser Federkraft kann das Gehäuse 1 in der Art eines Clips lösbar an dem durchbluteten Gewebe befestigt werden.

Die Lichtquelle 21 des Sensors 2 ist an der Außenseite der Deckplatte 12, die der Grundplatte 11 zugewandt ist, angeordnet, während der Detektor 22 an der Außenseite der Grundplatte 11, die der Deckplatte 12 zugewandt ist, angeordnet ist. Lichtquelle 21 und Detektor 22 liegen sich daher gegenüber.

Die Außenseiten des Gehäuses 1, die in Kontakt mit der Umgebung stehen, sind mit einem flexiblen, autoklavierbaren Material beschichtet. Im einzelnen sind dies die Grundplatte 11, die Deckplatte 12 sowie die Seitenelemente. Ebenso ist das Federelement 13 mit dem flexiblen, autoklavierbaren Material beschichtet, wenn sie nicht vollständig von dem Gehäuse 1 umschlossen und damit der Umgebung ausgesetzt sind.

Alternativ können alle Elemente des Gehäuses, also die Grundplatte 11, die Deckplatte 12, die Seitenelemente und das Federelement 13 vollständig aus dem flexiblen, autoklavierbaren Material gebildet sein.

## Patentansprüche

1. Vorrichtung zur Messung der Sauerstoffsättigung im Blut, umfassend ein Gehäuse (1), einen Sensor (2), ein Verbindungskabel (3) und einen Anschlußstecker (4), wobei
- das Gehäuse (1) eine Ausnehmung (5) zur Aufnahme von durchblutetem Gewebe eines Patienten aufweist;
- der Sensor (2), der an dem Gehäuse angeordnet ist und zumindest eine Lichtquelle (21) zur Emission von Licht, das das durchblutete Gewebe durchdringt, und einen Detektor (22) zum Empfang des durch das durchblutete Gewebe hindurchdringenden Lichtes aufweist, wobei es sich bei der Lichtquelle (21) und dem Detektor (22) um optische Halbleiter-Bauelemente handelt; und
- die Vorrichtung über den Anschlußstecker (4) mit einer Auswerteeinheit verbunden werden kann;
**dadurch gekennzeichnet, daß**
- das Gehäuse (1), der Sensor (2), das Verbindungskabel (3) und der Anschlußstecker (4) schlüssig und unlösbar miteinander verbunden sind;
- die Außenseiten zumindest des Gehäuses (1), des Verbindungskabels (3) und des Anschlußsteckers (4) aus einem ersten flexiblen, autoklavierbaren Material gebildet sind; und
- Lichtquelle (21) und Detektor (22) so ausgewählt sind, dass sie die benötigte Temperaturstabilität aufweisen, um den hohen Temperaturen beim Autoklavieren standzuhalten.

2. Vorrichtung nach Anspruch 1, **dadurch gekennzeichnet, daß** das Gehäuse (1) vollständig aus dem ersten flexiblen, autoklavierbaren Material gebildet ist.

3. Vorrichtung nach Anspruch 1, **dadurch gekennzeichnet, daß** das Gehäuse (1) eine Beschichtung aus dem ersten flexiblen, autoklavierbaren Material aufweist, wobei die Beschichtung die Außenseite des Gehäuses (1) bildet.

4. Vorrichtung nach einem der vorstehenden Ansprüche, **dadurch gekennzeichnet, daß** die Außenseiten des Sensors (2) aus einem zweiten autoklavierbaren Material bestehen, wobei das zweite autoklavierbare Material dasselbe oder ein anderes als das erste flexible, autoklavierbare Material sein kann.

5. Vorrichtung nach einem der vorstehenden Ansprüche, **dadurch gekennzeichnet, daß** das Verbindungskabel (3) eine Isolierung aus dem ersten flexiblen, autoklavierbaren Material aufweist.

6. Vorrichtung nach einem der vorstehenden Ansprüche, **dadurch gekennzeichnet, daß** der Sensor (2) in das Gehäuse (1) integriert ist, wobei die dem durchbluteten Gewebe zugewandten Außenseiten des Sensors (2) von dem Gehäuse gebildet werden.

7. Vorrichtung nach einem der vorstehenden Ansprüche, **dadurch gekennzeichnet, daß** das Gehäuse (1) eine Grundplatte (11) und eine Deckplatte (12), die von der Grundplatte (11) beabstandet ist und in einer Ebene, die parallel zu der Grundplatte (11) verläuft, verdrehbar ist, aufweist, wobei
- an den Längsseiten der Grundplatte (11) ein erstes Seitenelement (13) und ein zweites Seitenelement (14) angeordnet sind, während an einer Schmalseite der Grundplatte (11) ein drittes Seitenelement angeordnet ist:
- sich die Seitenelemente senkrecht zur Grundplatte (11) bis zur Deckplatte (12) erstrecken und gemeinsam mit der Grundplatte (11) und der Deckplatte (12) die Ausnehmung (5) zur Aufnahme des durchbluteten Gewebes umgrenzen, wobei die Ausnehmung (5) nach unten hin von der Grundplatte (11) und nach oben hin von der Deckplatte (12) abgeschlossen ist; und
- das Gehäuse (1) ein Federelement (13) aufweist, das an dem dritten Seitenelement und an der Grundplatte (11) anderseits befestigt ist, wobei das Federelement (13) die Grundplatte (11) in Richtung der Deckplatte (12) spannt.

8. Vorrichtung nach Anspruch 7, **dadurch gekennzeichnet, daß** die Lichtquelle (21) des Sensors (2) an der Außenseite der Deckplatte (12), die der Grundplatte (11) zugewandt ist, angeordnet ist und der Detektor (22) an der Außenseite der Grundplatte (11), die der Deckplatte (12) zugewandt ist, angeordnet ist.

9. Vorrichtung nach einem der vorstehenden Ansprüche, **dadurch gekennzeichnet, daß** das erste flexible, autoklavierbare Material ein Silicon ist.

10. Vorrichtung nach einem der vorstehenden Ansprüche, **dadurch gekennzeichnet, daß** das zweite autoklavierbare Material ein Silicon ist.

11. Verfahren zur Herstellung einer Vorrichtung nach einem der Ansprüche 1 bis 10, umfassend
- das Bereitstellen eines Gehäuses (1), eines Sensors (2), eines Verbindungskabels (3) und eines Anschlußsteckers (4), wobei zumindest einer dieser Bestandteile eine Außenseite aus einem nicht autoklavierbaren Material aufweist;
- das Besprühen der Außenseiten der Bestandteile mit einer autoklavierbaren Substanz;
wobei die autoklavierbare Substanz eine gel- oder schaumartige Konsistenz aufweist oder sich im gasförmigen Zustand befindet, so daß die flexible, autoklavierbare Substanz in die Oberflächen der Bestandteile unter Ausbildung der Außenseiten aus einem flexiblen, autoklavierbaren Material diffundiert.

12. Verfahren nach Anspruch 11, **dadurch gekennzeichnet, daß** die autoklavierbare Substanz eine Beschichtung aus einem flexiblen, autoklavierbaren Material bildet.

13. Verwendung einer Vorrichtung nach einem der Ansprüche 1 bis 10 zur Messung der Sauerstoffsättigung im Blut.

14. Verwendung nach Anspruch 13 zur Messung der Sauerstoffsättigung SₚO₂.

15. Verwendung nach Anspruch 13 oder Anspruch 14 zur Überwachung der Sauerstoffsättigung.

## Claims

1. Apparatus for measuring blood oxygen saturation, comprising a housing (1), a sensor (2), a connecting cable (3) and a plug (4), wherein
- the housing (1) has a cavity (5) for accepting a patient's tissue which is supplied with blood;
- the sensor (2) which is arranged on the housing and has at least one light source (21) for the emission of light which passes through the tissue which is supplied with blood and a detector (22) for receiving the light passing through the tissue which is supplied with blood, wherein the light source (21) and the detector (22) are optical semi-conductor components; and
- the apparatus can be connected to an evaluation unit via the plug (4);
**characterized in that**
- the housing (1), the sensor (2), the connecting cable (3) and the plug (4) are connected to one another in an interlocking and nondetachable manner;
- the outsides of at least the housing (1), of the connecting cable (3) and of the plug (4) are formed from a first flexible, autoclavable material; and
- the light source (21) and the detector (22) are selected such that they have the required temperature stability in order to resist the high temperatures during autoclaving.

2. Apparatus according to Claim 1, **characterized in that** the housing (1) is formed completely from the first flexible, autoclavable material.

3. Apparatus according to Claim 1, **characterized in that** the housing (1) has a coating comprising the first flexible, autoclavable material, the coating forming the outside of the housing (1).

4. Apparatus according to any of the preceding claims, **characterized in that** the outsides of the sensor (2) consist of a second autoclavable material, it being possible for the second autoclavable material to be the same as the first flexible, autoclavable material or to be a material other than the first flexible, autoclavable material.

5. Apparatus according to any of the preceding claims, **characterized in that** the connecting cable (3) has an insulation comprising the first flexible, autoclavable material.

6. Apparatus according to any of the preceding claims, **characterized in that** the sensor (2) is integrated into the housing (1), the outsides of the sensor (2) which face the tissue which is supplied with blood being formed by the housing.

7. Apparatus according to any of the preceding claims, **characterized in that** the housing (1) has a base plate (11) and a cover plate (12) which has a certain distance from the base plate (11) and is rotatable in a plane which is parallel to the base plate (11), wherein
- a first side element (13) and a second side element (14) are arranged on the longitudinal sides of the base plate (11) while a third side element is arranged on the narrow side of the base plate (11);
- the side elements extend perpendicularly to the base plate (11) up to the cover plate (12) and, together with the base plate (11) and the cover plate (12), bound the cavity (5) for accepting the tissue which is supplied with blood, the cavity (5) being closed at the bottom by the base plate (11) and at the top by the cover plate (12); and
- the housing (1) has a spring element (13) which is fastened to the third side element and to the base plate (11) on the other hand, the spring element (13) clamping the base plate (11) in the direction of the cover plate (12).

8. Apparatus according to Claim 7, **characterized in that** the light source (21) of the sensor (2) is arranged on the outside of the cover plate (12), which faces the base plate (11), and the detector (22) is arranged on the outside of the base plate (11) which faces the cover plate (12).

9. Apparatus according to any of the preceding claims, **characterized in that** the first flexible, autoclavable material is a silicone.

10. Apparatus according to any of the preceding claims, **characterized in that** the second autoclavable material is a silicone.

11. Method for producing an apparatus according to any of Claims 1 to 10, comprising
- the provision of a housing (1), a sensor (2), a connecting cable (3) and a plug (4), at least one of these parts having an outside comprising a non-autoclavable material;
- the spraying of the outsides of the parts with an autoclavable substance;
the autoclavable substance having a gel- or foam-like consistency or being present in the gaseous state so that the flexible, autoclavable substance diffuses into the surfaces of the parts with formation of the outsides comprising a flexible, autoclavable material.

12. Method according to Claim 11, **characterized in that** the autoclavable substance forms a coating comprising a flexible, autoclavable material.

13. Use of an apparatus according to any of Claims 1 to 10 for measuring blood oxygen saturation.

14. Use according to Claim 13 for measuring the oxygen saturation SₚO₂.

15. Use according to Claim 13 or Claim 14 for monitoring the oxygen saturation.

## Revendications

1. Dispositif pour mesurer la saturation en oxygène dans le sang, comprenant un boîtier (1), un capteur (2), un câble de raccordement (3) et une fiche de connexion (4), dans lequel
- le boîtier (1) présente un évidement (5) pour recevoir le tissu vascularisé d'un patient ;
- le capteur (2) est agencé sur le boîtier et présente au moins une source de lumière (21) pour une émission de lumière, qui traverse le tissu vascularisé, et un détecteur (22) pour recevoir la lumière traversant le tissu vascularisé, dans lequel il s'agit, dans le cas de la source de lumière (21) et du détecteur (22), de composants semi-conducteurs optiques ; et
- le dispositif peut être raccordé via la fiche de connexion (4) à une unité d'exploitation ;
**caractérisé en ce que**
- le boîtier (1), le capteur (2), le câble de raccordement (3) et la fiche de connexion (4) sont raccordés l'un à l'autre de manière pertinente et non détachable ;
- les faces externes au moins du boîtier (1), du câble de raccordement (3) et de la fiche de connexion (4) sont formées à partir d'un premier matériau flexible à même d'être traité à l'autoclave ; et
- la source de lumière (21) et le détecteur (22) sont choisis de sorte qu'ils présentent la stabilité à la température nécessaire pour résister aux températures élevées lors d'un traitement à l'autoclave.

2. Dispositif selon la revendication 1, **caractérisé en ce que** le boîtier (1) est formé complètement à partir du premier matériau flexible à même d'être traité à l'autoclave.

3. Dispositif selon la revendication 1, **caractérisé en ce que** le boîtier (1) présente un revêtement constitué du premier matériau flexible à même d'être traité à l'autoclave, le revêtement formant la face externe du boîtier (1).

4. Dispositif selon l'une quelconque des revendications précédentes, **caractérisé en ce que** les faces externes du capteur (2) se composent d'un second matériau à même d'être traité à l'autoclave, le second matériau à même d'être traité à l'autoclave pouvant être le même matériau ou un autre matériau que le premier matériau flexible à même d'être traité à l'autoclave.

5. Dispositif selon l'une quelconque des revendications précédentes, **caractérisé en ce que** le câble de raccordement (3) présente une isolation à partir du premier matériau flexible à même d'être traité à l'autoclave.

6. Dispositif selon l'une quelconque des revendications précédentes, **caractérisé en ce que** le capteur (2) est intégré au boîtier (1), dans lequel les faces externes, tournées vers le tissu vascularisé, du capteur (2) sont formées par le boîtier.

7. Dispositif selon l'une quelconque des revendications précédentes, **caractérisé en ce que** le boîtier (1) présente une plaque de base (11) et une plaque de recouvrement (12), qui est à une certaine distance de la plaque de base (11) et qui peut tourner dans un plan s'étendant parallèlement à la plaque de base (11), dans lequel
- un premier élément latéral (13) et un deuxième élément latéral (14) sont agencés sur les faces longitudinales de la plaque de base (11), tandis qu'un troisième élément latéral est agencé sur un petit côté de la plaque de base (11) ;
- les éléments latéraux s'étendent perpendiculairement à la plaque de base (11) jusqu'à la plaque de recouvrement (12) et délimitent, conjointement avec la plaque de base (11) et la plaque de recouvrement (12), l'évidement (5) permettant de recevoir le tissu vascularisé, l'évidement (5) étant isolé, vers le bas, de la plaque de base (11) et, vers le haut, de la plaque de recouvrement (12) ; et
- le boîtier (1) présente un élément à ressort (13), qui est fixé sur le troisième élément latéral et, par ailleurs, sur la plaque de base (11), l'élément à ressort (13) serrant la plaque de base (11) en direction de la plaque de recouvrement (12).

8. Dispositif selon la revendication 7, **caractérisé en ce que** la source de lumière (21) du capteur (2) est agencée sur la face externe de la plaque de recouvrement (12) qui est tournée vers la plaque de base (11), et **en ce que** le détecteur (22) est agencé sur la face externe de la plaque de base (11) qui est tournée vers la plaque de recouvrement (12).

9. Dispositif selon l'une quelconque des revendications précédentes, **caractérisé en ce que** le premier matériau flexible à même d'être traité à l'autoclave est une silicone.

10. Dispositif selon l'une quelconque des revendications précédentes, **caractérisé en ce que** le second matériau à même d'être traité à l'autoclave est une silicone.

11. Procédé pour fabriquer un dispositif selon l'une quelconque des revendications 1 à 10, comprenant:
- la préparation d'un boîtier (1), d'un capteur (2), d'un câble de raccordement (3) et d'une fiche de connexion (4), dans lequel au moins l'un de ces composants présente une face externe constituée d'un matériau qui ne peut être traité à l'autoclave ;
- la pulvérisation des faces externes des composants avec une substance à même d'être traitée à l'autoclave ;
dans lequel la substance à même d'être traitée à l'autoclave présente une consistance de type gel ou de type mousse, ou se présente à l' état gazeux de sorte que la substance flexible à même d'être traitée à l'autoclave diffuse dans les surfaces des composants en formant les faces externes à partir d'un matériau flexible à même d'être traité à l'autoclave.

12. Procédé selon la revendication 11, **caractérisé en ce que** la substance à même d'être traitée à l'autoclave forme un revêtement à base d'un matériau flexible à même d'être traité à l'autoclave.

13. Utilisation d'un dispositif selon l'une quelconque des revendications 1 à 10, pour mesurer la saturation en oxygène dans le sang.

14. Utilisation selon la revendication 13, pour mesurer la saturation en oxygène de type SₚO₂.

15. Utilisation selon la revendication 13 ou 14, pour surveiller la saturation en oxygène.
